# EUROPEAN PATENT SPECIFICATION

(11) **EP 3 948 936 B1**
(45) Date of publication and mention of the grant of the patent: **20.03.2024**
(21) Application number: 20781932.7
(22) Date of filing: 03.04.2020
(51) Int. Cl.: A61N 1/05, A61N 1/04, A61N 1/36, A61N 1/372

(54) **METHOD FOR MANUFACTURING AN ELECTRODE ARRAY FOR A NEUROPROSTHETIC DEVICE**
VERFAHREN ZUR HERSTELLUNG EINES ELEKTRODENARRAYS FÜR EINE NEUROPROTHETISCHE VORRICHTUNG
PROCÉDÉ DE FABRICATION DE RÉSEAU D'ÉLECTRODES POUR DISPOSITIF NEUROPROTHÉTIQUE

(30) Priority: 05.04.2019 US 201962830347 P
(43) Date of publication of application: 09.02.2022
(73) Proprietor: Memstim, LLC, Louisville KY 40202-2654 (US)
(72) Inventor: JOHNSON, Angelique Candace, Louisville, Kentucky 402022654 (US); STROTMAN, Lindsay Nicole, Louisville, Kentucky 402022654 (US)
(74) Representative: LKGlobal UK Ltd.
(86) International application number: PCT/US2020/026549
(87) International publication number: WO 2020/206230

(56) References cited:
- WO-A2-2018/098046
- US-A1- 2002 074 587
- US-A1- 2003 233 133
- US-A1- 2006 227 277
- US-A1- 2006 227 277
- US-A1- 2010 229 942
- US-A1- 2011 180 305
- US-A1- 2013 023 816
- US-A1- 2013 023 816
- US-A1- 2013 337 604
- XIAOWEI YU ET AL: "Materials, Processes, and Facile Manufacturing for Bioresorbable Electronics: A Review", ADVANCED MATERIALS, VCH PUBLISHERS, DE, vol. 30, no. 28, 7 May 2018 (2018-05-07), page n/a, XP071872712, ISSN: 0935-9648, DOI: 10.1002/ADMA.201707624

## Description

### FIELD OF THE DISCLOSURE

The present disclosure relates generally to electrode arrays and, more particularly, to a method of manufacturing an electrode array for a neuroprosthetic device.

### BACKGROUND

Neuroprosthetic devices are often used for treating neurological diseases and disorders such as Parkinson's disease, deafness, epilepsy, chronic pain, paralysis, and blindness. One example of a neuroprosthetic device is a cochlear implant, a medical device implanted into the cochlea of an organism. The cochlear implant includes a stimulator that generates electrical signals and an electrode array that provides the electrical signals to nerve fibers in the cochlea to improve hearing.

Electrode arrays have been manufactured using a variety of manufacturing techniques, including microfabrication processes and certain direct write printing techniques. In some instances, the electrode arrays are manufactured by hand. Microfabrication processes use vapor and/or electro deposition of materials and subtractive techniques, such as etching and machining, may then be used during microfabrication to create three-dimensional (3D) features including traces, electrode contacts, channels, etc. In some instances, the 3D features formed using subtractive techniques are poorly defined. Additionally, the deposition processes tend to use undesirable gases and the materials used during microfabrication tend to be more rigid and are unable to withstand common flexing, bending, and/or pulling.

Screen printing is another microfabrication technique often used to produce electrode arrays. Although useful for printing a large range of materials, screen printing requires the use of a mask, requires direct contact with a flat substrate, and is limited when forming small 3D features including traces, electrode contacts, channels, etc.

Inkjet printing is a form of direct write printing and involves extruding a material, such as an ink, through a nozzle as the nozzle moves across the substrate to form 3D features onto the substrate. Although suitable for printing electronics on simple planar substrates, inkjet printing tends to be insufficient for use in printing curved electrode arrays, incapable of printing line widths smaller than 30 µm with adequate resolution, and incapable of printing densely weighted material limiting type and/or content of metal particles to be printed. Additionally, inkjet printing tends to be incompatible with high viscosity silicones and fluoropolymers frequently used as structural and insulating layers in electrode arrays. As such, another technique is typically required to deposit high viscosity material to form the structural or insulating layers of an electrode array.

Aerosol jet printing is another form of direct write printing and involves propelling a jet stream of material onto the substrate. As aerosol jet printing is an aerodynamic stream-based technology, aerosol jet printing can produce electrode array line widths as small as 5 µm. Additionally, the print head of an aerosol jet printer allows for multi-axis rotation enabling printing on curved substrates. However, aerosol jet printing tends to be incapable of printing various nonconductive materials including insulating and/or high viscosity materials, and tends to print discontinuous lines which can adversely affect electrical continuity conductive features of the electrode array.

For instance, US 2006/227277 A1 discloses preparing a contact spot electrode of liquid crystal display, ba means of producing contact holes in insulating-/passivating layer, applying photo resist on substrate and producing gate-/data contact spot electrode by incinerating photo resist.

The present disclosure is aimed at solving the problems identified above.

### SUMMARY

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes.

The present disclosure provides a method of manufacturing an electrode array. The method comprises the steps of: forming a first insulating layer from a first nonconductive material with said first insulating layer having opposed first and second surfaces; depositing, by pressure-driven extrusion printing, a first conductive material over a portion of the second surface of the first insulating layer to form a first conductive layer having opposed first and second surfaces with the first surface of the first conducting layer facing the first insulating layer, with another portion of the second surface of the first insulating layer remaining exposed; depositing a second nonconductive material over a portion of the second surface of the first conductive layer and over the exposed portion of the second surface of the first insulating layer to form a second insulating layer having opposed first and second surfaces and a gap extending between the first and second surfaces of the second insulating layer with the gap exposing a portion of the second surface of the first conductive layer; depositing, by pressure-driven extrusion printing, a second conductive material into the gap and over the exposed portion of the second surface of the first conductive layer to form a second conductive layer electrically connected to the first conductive layer to form at least one electrode to form the electrode array.

### BRIEF DESCRIPTION OF THE DRAWINGS

Advantages of the present invention will be readily appreciated, as the same becomes better understood by reference to the following detailed description, when considered in connection with the accompanying drawings.
Figure 1 is a semi-schematic, partial cross-sectional perspective view of a portion of an auditory system of an organism and a neuroprosthetic device implanted into the cochlea of the organism.
Figure 2 is a schematic perspective view of a segment of an electrode array for the neuroprosthetic device.
Figures 3A through 3F are cross-sectional schematic views of steps of a method of manufacturing the electrode array according to an embodiment of the present disclosure. Notably, the individual material layers of the electrode array and instrumentation used to form the electrode array are not drawn to scale.
Figure 4 is a cross-sectional schematic view of an alternative step of the method of manufacturing the electrode array illustrated in Figures 3A-3F. Notably, the individual material layers of the electrode array and the instrumentation used to form the electrode array are not drawn to scale.
Figure 5 is a cross-sectional schematic view of another alternative step of the method of manufacturing the electrode array. Notably, none of the features or individual material layers are drawn to scale.

### DETAILED DESCRIPTION

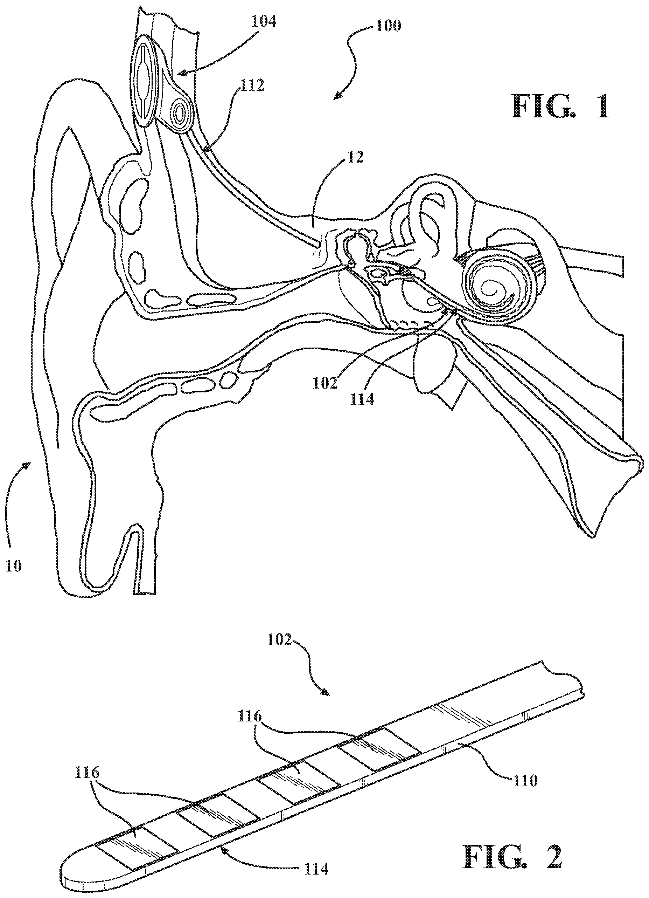

Referring now to the figures, where like numerals indicate like or corresponding parts throughout the several views, embodiments of a method of manufacturing an electrode array 102 are described in detail below. The electrode array 102 formed by the method of the present disclosure may be used in a neuroprosthetic device for treating various neurological diseases and disorders. Non-limiting examples of neuroprosthetic devices include deep brain stimulators, spinal cord stimulators, retinal prostheses, cochlear prostheses, and the like. For example, and as shown in Figure 1, the electrode array 102 may be used for a cochlear prosthesis 100, a neuroprosthetic device implantable into the cochlea 12 of an organism 10 (such as a human or an animal) for treating hearing loss. Due, at least in part, to the presence of a flexible material, such as silicone, in the individual material layers, the electrode array 102 formed by the method of the present disclosure is flexible and elastic and can be readily implanted into small, curved spaces, such as the cochlea 12 of the organism 10. It should be appreciated that the electrode array 102 could be used for any suitable stimulating device, not limited to neuroprosthetic devices. Applications of the electrode array 102 outside of medical devices are also contemplated.

For medical applications, the electrode array 102 is desirably formed from biocompatible materials, i.e., materials that are friendly and unharmful to living tissue. In the present disclosure, the electrode array 102 formed by the method includes biocompatible materials, rendering the electrode array 102 as being a biocompatible neuroprosthetic device.

Figure 2 is a schematic representation of the prosthesis 100 including the electrode array 102. As shown in Figures 1 and 2, the electrode array 102 has an interconnect region 112 and an electrode region 114. The interconnect region 112 allows for electrical connection of the electrode array 102 to an external device, such as a stimulator 104 of the neuroprosthetic device 100 as shown in Figure 1. The electrode region 114 includes a substrate 110 supporting a plurality of electrodes 116 of the electrode array 102 as best shown in Figure 2. Although four electrodes 116 are illustrated in Figure 2, it should be appreciated that the electrode array 102 may have any number of electrodes 116. In cochlear prostheses, for example, the stimulator 104 operates to produce electrical signals and the electrodes 116 operate to pass the electrical signals to the cochlea 12 of the organism 10 to treat hearing loss. The stimulator 104 and the electrode array 102 are connected by a connector, such as a feedthrough pin. The connector is connected to the electrodes 116 of the electrode array 102 via a conductive wire, for example, embedded in the substrate 110. Further details of the electrode array 102 are identified below in connection with the detailed description of a method of manufacturing the electrode array 102.

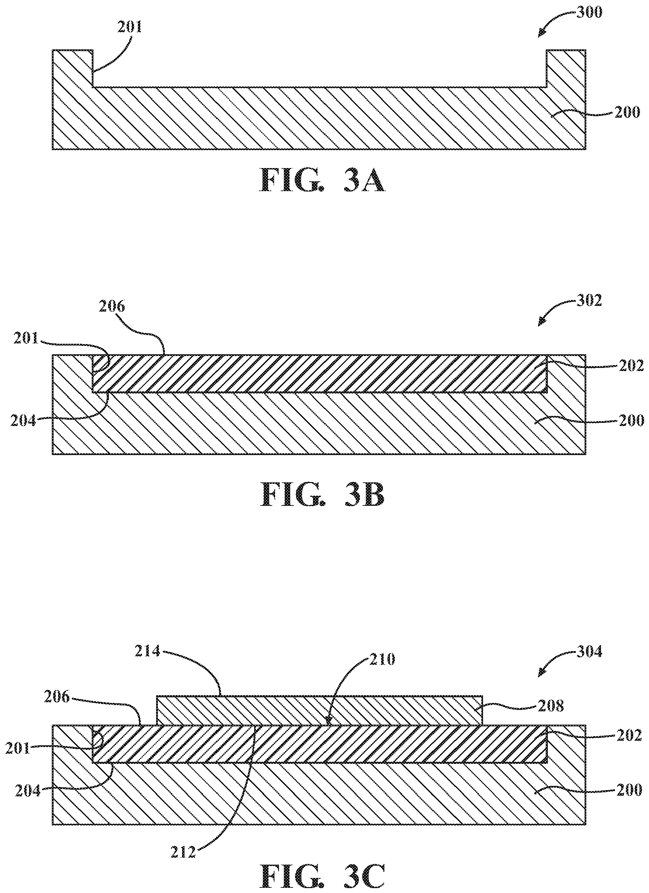

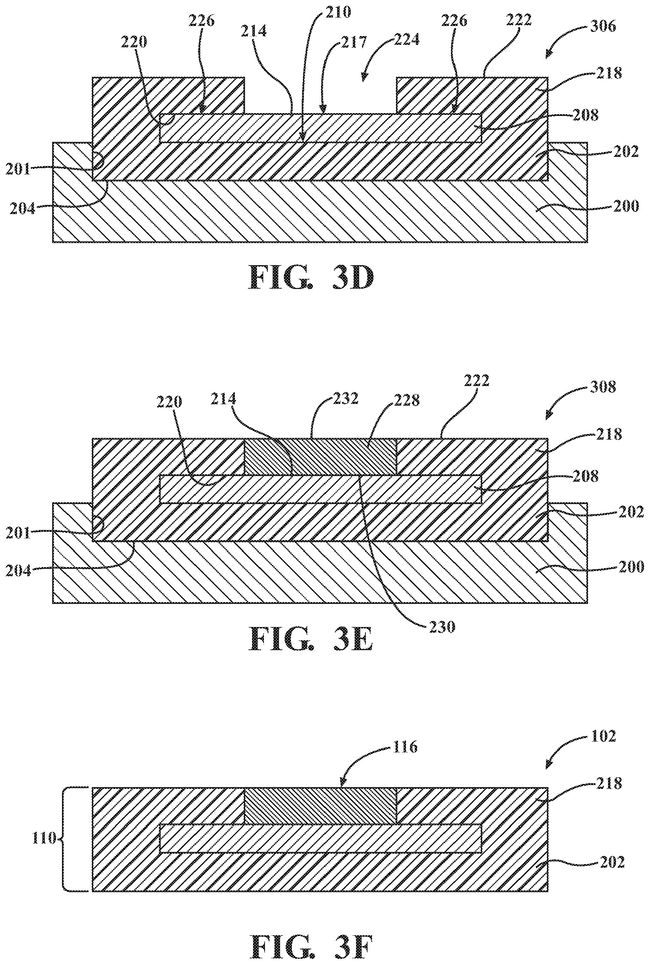

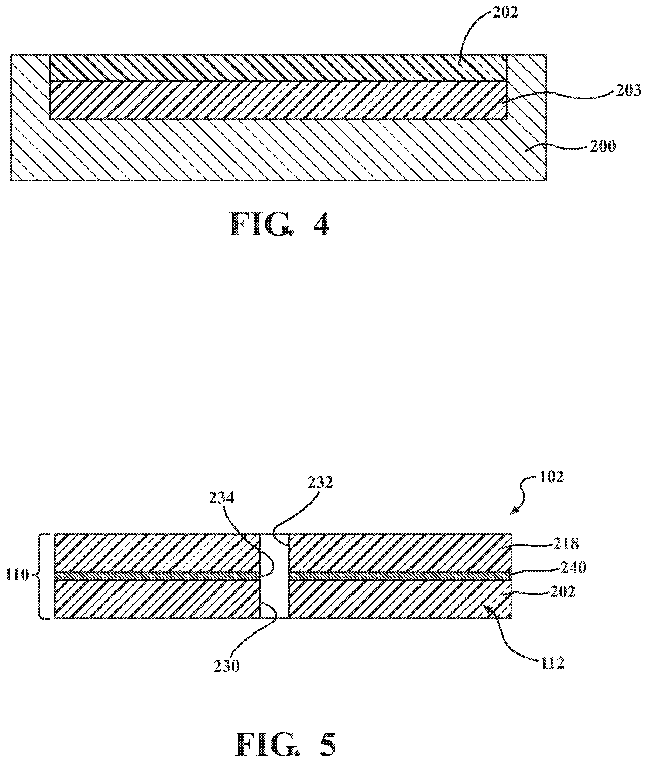

Details of the method of manufacturing the electrode array 102 are described below with reference to Figures 3 and 4. As shown in Figure 3A, the method includes the step of providing a mold (step 300). The mold typically includes opposing top and bottom parts, with the bottom part 200 of the mold schematically illustrated in Figure 3A. In the illustrated embodiment, the bottom part 200 of the mold defines a cavity 201. As described below, the electrode array 102 is built within the cavity 201 of the mold 200.

Referring to Figure 3B, the method includes the step of forming a first insulating layer 202 from a first nonconductive material with said first insulating layer 202 having opposed first 204 and second 206 surfaces. In an embodiment, the forming step is accomplished by depositing the first nonconductive material into the cavity 201 of the bottom part 200 of the mold to form the first insulating layer 202 with the first surface 204 of the first insulating layer 202 facing the bottom part 200 of the mold (step 302). The first insulating layer 202 forms part of the substrate 110 of the electrode array 102 shown in Figure 2.

The first nonconductive material is an electrically insulating material and is biocompatible. In an embodiment, the first nonconductive material has a resistivity of at least 1x1011 ohm·m. Non-limiting examples of biocompatible nonconductive materials include silicones, fluoropolymers (such as polytetrafluoroethylene), oxides, pre-stretched elastomers, poly(p-xylene), polyimides, polyurethanes, and combinations thereof. In a particular embodiment, the nonconductive material is a silicone or a combination of silicones. In an alternative embodiment, the nonconductive material could be a silicone(s) in combination with another biocompatible nonconductive material. Use of biocompatible nonconductive materials for the first conductive material other than silicone is also contemplated.

The first insulating layer 202 may have any suitable thickness. For example, the first insulating layer 202 may have a thickness suitable for providing a support for building subsequent layers of the electrode array 102. In an embodiment, the first insulating layer 202 has a thickness of up to about 250 µm. In another embodiment, the thickness of the first insulating layer 202 is from about 10 to about 250 µm. In another embodiment, the thickness of the first insulating layer 202 is from about 10 to about 60 µm. In yet another embodiment, the thickness of the first insulating layer 202 is from about 10 to about 40 µm.

The first nonconductive material is deposited utilizing any suitable additive manufacturing process or technique. In an embodiment, the depositing of the first nonconductive material is performed by printing. For example, the bottom part 200 of the mold may be transferred to a print bed of a suitable printer, and the first nonconductive material is printed into the cavity 201 of the bottom part 200 of the mold. In an embodiment, the depositing of the first nonconductive material is accomplished utilizing pressure-driven extrusion printing, which is a form of direct write printing to form the first insulating layer 202. In another embodiment, depositing of the first nonconductive material is performed by three-dimensional printing, an additive manufacturing process where material is deposited, in single or multiple layers, into the cavity 201 of the bottom part 200 of the mold. In an embodiment, a three-dimensional (3D) construct including one or more 3D features may be formed by successively adding layers of the first nonconductive material through multiple passes of the printer.

In an embodiment, the printing of the first nonconductive material is performed utilizing an nScrypt^{®} 3Dn printer available from nScrypt Inc. (Orlando, FL). The nScrypt^{®} printer is a pressure-driven extrusion printing device, where the first nonconductive material is extruded through a print nozzle of the printer in a liquid state. The printer includes at least one print head defining the print nozzle. In an embodiment, the printer has first and second print heads with each print head defining a nozzle. The nozzle of the first print head is configured to extrude nonconductive material(s) for forming first 202 and second 218 insulating layers of the electrode array 102. The nozzle of the second print head is configured to extrude conductive material(s) for forming first 208 and second 228 conductive layers of the electrode array 102 described below. Each of the nozzles has an orifice of from about 10 to 500 µm in diameter. In an embodiment, the nozzles of the printer have an orifice of from about 10 to about 125 µm. Additionally, the nozzles of the printer may have any suitable configuration. In an embodiment, the orifice of the nozzle for the nonconductive materials is smaller than the orifice of the nozzle used for the conductive materials. For example, the orifice of the nozzle for the nonconductive materials may be about 75 µm in diameter, and the orifice for the conductive materials may be about 125 µm in diameter.

Because silicone has room temperature cure times of several hours, printing embodiments of the first nonconductive material including silicone often poses a challenge. With reference to Figure 4, one way to overcome this challenge is to form a nonconductive support 203 having a desired configuration, and then build the electrode array 102 on the support 203. The support 203 is formed from a nonconductive material, such as the first nonconductive material used to form the insulating layer 202. The support 203 may have any suitable configuration and size and functions as a platform upon which the electrode array 102 is built. As described further below, the support 203 may be removed from the electrode array 102 once the electrode array 102 has been built or formed.

The support 203 may be formed when the electrode array 102 is built. For example, and with continued reference to Figure 4, the method includes the steps of depositing a first portion of the first nonconductive material by any suitable additive manufacturing process to form the support 203, and then depositing a second portion of the first nonconductive material over the deposited first portion by printing to form the first insulating layer 202. In an embodiment, the first portion of the first nonconductive material to form the support 203 is also deposited by printing. Typically, the first portion of the first nonconductive material is larger than the second portion of the first nonconductive material. In other words, a bulk or majority of the nonconductive material is deposited to form the support 203, and then the remainder of the nonconductive material is deposited over the first portion to form the first insulating layer 202 of the electrode array 102. During the second part, at least one layer of the remainder of the nonconductive material may be printed by at least one pass of the printer. In an embodiment, depositing (such as printing) of the first and second portions of the first nonconductive material is accomplished at any suitable rate, such as at a rate of about 10 to about 20 mm/s. Depositing of the first nonconductive material may also be accomplished at a pressure of less than 60 psi.

Other ways of overcoming the challenge for printing silicone, such as heating the printer to cure the first nonconductive material when printed or printing directly into the cavity 201 of a curved bottom part 200 of the mold to prevent fluid flow before curing, are also contemplated.

Once the first and second parts of the depositing step 302 are complete, the method further includes the step of curing the first and second portions of the first nonconductive material during or after the step of depositing the second portion of the first nonconductive material to solidify the support 203 and the first insulating layer 202. The first and second portions may be cured simultaneously or separately. Curing may be accomplished utilizing a hot plate or an oven at any suitable curing temperature for a predetermined period of time. For silicone, curing may be accomplished, for example, at a temperature up to about 160°C for about 1 to about 15 minutes. Alternatively, curing may be accomplished during printing of the first and second portions of the nonconductive material.

Notably, step 302 shown in Figure 3B illustrates the embodiment where the electrode array 102 is built inside the cavity 201 of the bottom part 200 of the mold without forming a support 203. In this embodiment, all of the first nonconductive material deposited into the cavity 201 of the bottom part 200 of the mold is used to form the first insulating layer 202.

In an embodiment, the method includes the step of forming a nonconductive ink including the first nonconductive material and a curing agent. In an embodiment, the nonconductive ink further includes a solvent (such as, but not limited to, xylene, toluene, ligroin, mineral spirits, chlorinated hydrocarbons, and combinations thereof) and optionally one or more additives. In the embodiment illustrated in Figure 3B, the step of forming the first insulating layer includes printing the nonconductive ink into the cavity 201 of the bottom part 200 of the mold utilizing a suitable printer, such as a pressure-driven extrusion printer. In the embodiment illustrated in Figure 4, the step of depositing the second portion of the first nonconductive material includes depositing the nonconductive ink over the deposited first portion of the first nonconductive material by printing to form the first insulating layer 202. During printing, the nonconductive ink is printed onto the second segment 210 utilizing the pressure-driven extrusion printer described above.

In an alternative embodiment, the step of depositing the first nonconductive material to form the first insulating layer 202 could be accomplished utilizing other additive manufacturing processes.

In an embodiment, the first insulating layer 202 may include one or more 3D features, such as a channel(s) or other structure to aid in surgical placement or implantation of the prothesis into the organism 10. Additionally, the second surface 206 of the first insulating layer 202 may be roughened to improve adhesion of subsequently formed layers (such as the first conductive layer 208 of the electrode array 102). Other features may include a wavy surface contour to enhance the ability of the electrode array 102 to stretch without adversely affecting the performance of conductive features of the electrode array 102 and/or the first insulating layer 202 may be pre-stretched to enhance the elasticity of the electrode array 102. These features may be obtained during printing of the first nonconductive material, such as by utilizing a mapping feature of the printer.

With reference to Figure 3C, the method further includes the step of depositing, by pressure-driven extrusion printing, a first conductive material over a portion 210 of the second surface 206 of the first insulating layer 202 to form a first conductive layer 208 having opposed first 212 and second 214 surfaces with the first surface 212 of the first conductive layer 208 faces the first insulating layer 202 and another portion 216 of the second surface 206 of the first insulating layer 202 remaining exposed (step 304). In an embodiment, the printing of the first conductive material is performed utilizing the nScrypt^{®} printer described above, where the first conductive material is printed through the second nozzle. Again, the orifice of the nozzle for the first conductive material is larger than the nozzle used for printing the nonconductive material. Although the nozzles may have any suitable configuration (as previously mentioned), the second nozzle for printing the first conductive material may have a chamfered configuration. The chamfered nozzle is desirable to prevent agglomeration of metal particles of the conductive material, thereby reducing waste.

The printing of the first conductive material to form the first conductive layer 208 may be accomplished at a rate of from about 1 to about 50 mm/s when printing through a nozzle having an orifice with a 75 µm diameter. In another embodiment, the printing of the first conductive material is accomplished at a rate of from about 1 to about 15 mm/s when printing through a nozzle having an orifice with a 75 µm diameter. The first conductive layer 208 formed may have any suitable thickness. In an embodiment, the thickness of the first conductive layer 208 is from about 10 to about 100 µm. In another embodiment, the thickness of the first conductive layer 208 is from about 10 to about 50 µm. In yet another embodiment, the thickness of the first conductive layer 208 is from about 20 to about 30 µm. Additionally, the printing of the first conductive layer 208 may be accomplished at a pressure of less than 100 psi.

In an embodiment, the step of depositing the first conductive material includes forming the first conductive layer 208 having a plurality of 3D conductive features. The 3D conductive features include, for example, electrode contacts, traces, or interconnects for connecting the electrode array 102 to the stimulator 104 by the connector 106. Surface mapping may be used to determine step heights and surface roughness of the surface to ensure the nozzle of the printer remains a consistent distance from the surface during printing.

The pressure-driven extrusion printing technique allows for the precise formation of the 3D conductive features. Additionally, the pressure-driven extrusion printing reduces time and costs by eliminating additional process steps, such as various subtractive manufacturing processes such as etching or machining to form the 3D conductive features. The pressure-driven extrusion printing may be repeated multiple times to form multiple layers of the conductive material that collectively form the first conductive layer 208 including one or more 3D conductive features.

In another embodiment, the method includes the step of patterning all but an exposed portion 217 of the second surface 214 of the first conductive layer 208 (which is described further in connection with step 306 and Figure 3D below) to form the plurality of 3D conductive features. Patterning may be accomplished utilizing any suitable patterning technique known in the industry, such as by a lift-off process using a photoresist. Further processing of the first conductive layer 208 utilizing one or more subtractive techniques may also be employed to further define the configuration of the 3D conductive features. Alternatively, microfabrication techniques such as chemical and/or physical deposition processes, may be used to define the configuration of the 3D conductive features. For example, laser etching may be used to form individual traces in the first conductive layer 208.

In an embodiment, the method includes the step of forming a first conductive ink including the first conductive material disposed in a vehicle. The conductive material of the first conductive ink includes electrically conductive particles that are also biocompatible. Typically, the electrically conductive particles have a resistivity of less than 1x10-7 ohm·m. Non-limiting examples of conductive particles include medical grade platinum particles, silver particles, copper particles, gold particles, chromium particles, titanium particles, iridium particles, stainless steel particles, conductive polymers, carbon nanotubes, and combinations thereof. Typically, the particles have an effective particle diameter of less than 1 µm and may be referred to as nanoparticles. In an embodiment, the particles have an effective particle diameter of from about 50 nm to about 1 µm. Alternatively, the particles could have an effective particle diameter up to about 10 µm. In an embodiment, the conductive material includes platinum particles, and the first conductive ink may be referred to as a platinum ink. In another embodiment, the platinum particles are optimized by including platinum particles of varying shapes, sizes, and/or concentrations to obtain superior electrical properties such as charge storage capacity, impedance, etc.

In an embodiment, the vehicle is or includes silicone, such as polydimethylsiloxane, which imparts elasticity to the first conductive ink. The silicone is typically used with a curing agent. In another embodiment, the vehicle further includes a siloxane and optionally one or more traditional solvents (such as toluene, heptane, etc.). The siloxane functions to reduce the viscosity of the first conductive ink and tends to evaporate slowly compared to traditional solvents, thereby maintaining ink consistency during printing. Non-limiting examples of suitable siloxanes for the first conductive ink include cyclic siloxanes (such as hexamethylcyclotrisiloxane, octamethylcyclotetrasiloxane, etc.) and linear siloxanes (such as hexamethyldisiloxane, octamethyltrisiloxane, etc.). One or more additives (such as surfactants etc.) may also be used in the first conductive ink.

The first conductive ink is formed by combining the conductive material, the silicone, the siloxane, and optionally the additive(s). Combining may be accomplished, for example, by mixing the conductive material, the silicone, and the siloxane (and additives) with sonication, planetary centrifugal mixing, roll-to-roll milling, and/or the like. Other methods of combining the components of the first conductive ink known in the art are also contemplated. Once the first conductive ink is formed, the step of depositing the first conductive material is further defined as depositing the first conductive ink over the portion 210 of the second surface 206 of the first insulating layer 202. In addition, the first conductive ink is cured, which may be accomplished during printing or after printing using an oven or the like.

In an embodiment, the formation of the 3D conductive features may be controlled during printing. One way of controlling the formation of the 3D conductive features is to control settings and/or usability of the printer. For example, the depositing of the first conductive material is formed utilizing the printer and includes the step of depositing the first conductive material onto the second surface 206 of the first insulating layer 202 with multiple passes, varying speeds, varying pressures, or combinations thereof of the printer to form the first conductive layer 208 having multiple thicknesses defining a plurality of the 3D conductive features. For example, 3D conductive features where an electrode contact is to be formed may be thicker than 3D conductive features in other locations such as 3D conductive features that extend to the stimulator 104 of the neuroprosthetic device 100. Additionally, 3D conductive features may be corrugated to enhance flexibility of the electrode array 102 without significant loss in conductivity. Another way of controlling the formation of the 3D conductive features is to control the composition of the first conductive ink. For example, during the depositing of the first conductive ink the method includes the step of varying a particle density, a viscosity, or combinations thereof of the first conductive ink to form the first conductive layer 208 having the multiple thicknesses defining the plurality of 3D conductive features.

With reference to Figure 3D, the method further includes the step of depositing a second nonconductive material over at least one portion 226 of the second surface 214 of the first conductive layer 208 and over the exposed portion(s) 216 of the second surface 206 of the first insulating layer 202 to form a second insulating layer 218 having opposed first 220 and second 222 surfaces and a gap 224 extending between the first 220 and second 222 surfaces (step 306). During step 306, the method includes embedding the portion 210 of the first conductive layer 208 between the second surface 206 of the first insulating layer 202 and the first surface 220 of the second insulating layer 218.

The gap 224 exposes the portion 217 of the second surface 214 of the first conductive layer 208. The portion 217 of the second surface 214 remains unpatterned. As described below, a second conductive layer 228 is deposited on the portion 217 of the second surface 214 of the first conductive layer 208 to form the electrode 116 of the electrode array 102.

In an embodiment, the second nonconductive material deposited during step 306 has the same composition as the first nonconductive material deposited during step 302. Additionally, the second nonconductive material may be deposited utilizing the same deposition technique as previously described for the deposition of the first nonconductive material to form the first insulating layer 202 in step 302. The second insulating layer 218 formed may have any suitable thickness. In an embodiment, the second insulating layer 218 has a thickness of up to about 250 µm. In another embodiment, the thickness of the second insulating layer 218 is from about 10 to about 250 µm. In another embodiment, the thickness of the second insulating layer 218 is from about 10 to about 60 µm. In yet another embodiment, the thickness of the second insulating layer 218 is from about 10 to about 50 µm. In addition, the thickness of the second insulating layer 218 may be the same as or different from the thickness of the first insulating layer 202. The combination of the first 202 and second 218 insulating layers form the substrate 110 and is adapted to electrically insulate the various conductive elements of the electrode array 102. Additionally, the second surface 222 of the second insulating layer 218 serves as a support surface for building additional conductive elements (in a vertical direction) of the electrode array 102.

In an embodiment, and with reference to Figure 5, the method includes the steps of forming at least one aperture 230 in the first insulating layer 202 and forming at least one aperture 232 in the second insulating layer 218. The apertures 202, 232 are typically formed in the interconnect region 112 of the electrode array 102. In an embodiment, the electrode array 102 further includes a layer 240 of conductive material between the first 202 and second 218 insulating layers. The layer 240 includes conductive traces and/or other features that connect or attach to the electrode(s) 116. Accordingly, and in this embodiment, the method includes the steps of forming at least one aperture 230 in the first insulating layer 202, forming at least one aperture 234 in the layer 240, and forming at least one aperture 232 in the second insulating layer 218 The at least one aperture 232 of the second insulating layer 218 is aligned with the at least one aperture 230 of the first insulating layer 202 (and aligned with the at least one aperture 234 of the layer 240). The aligned apertures 230, 232 (and 234) are adapted to receive a connector, such as a feedthrough pin, of the stimulator 106 of the neuroprosthetic device 100. The forming steps are accomplished utilizing the printing described above for depositing the first and second nonconductive materials.

In an embodiment, the step of forming the at least one aperture 230 in the first insulating layer 202 is accomplished during the step of depositing the first nonconductive material. The step of forming the at least one aperture 232 in the second insulating layer 218 is accomplished during the step of depositing the second nonconductive material. Additionally, the step of forming the at least one aperture 234 in the layer 240 is accomplished during depositing of the first conductive material. The depositing steps utilize, for example, the pressure-driven extrusion printing as described above. Alternatively, the apertures 230, 232 (and 234) may be formed utilizing a suitable subtractive technique, such as by forming the apertures 230, 232 (and 234) utilizing a suitable poking or punching tool or device. Alternatively, conventional techniques for attaching the electrode array 102 to the stimulator 104, such as by wire-bonding or welding, could also be used.

Once the connector(s) has/have been disposed or inserted in the aligned apertures 230, 232 (and 234), the conductive ink may be introduced into the apertures 230, 232 (and 234) to form an electrical connection between the electrode array 102 and the stimulator 104. Additional layers of the conductive ink may also be added to further secure the electrical connection.

Referring to Figure 3E, the method further includes the step of depositing a second conductive material into the gap 224 and over the exposed portion 217 of the second surface 214 of the first conductive layer 208 to form the second conductive layer 228 electrically connected to the first conductive layer 208 (step 308). The depositing of the second conductive material into the gap 244 is accomplished utilizing pressure-driven extrusion printing utilizing a pressure-driven extrusion printer, such as the nScrypt^{®} described above. The second conductive layer 228 touches and/or is in direct contact with the first conductive layer 208 to establish the electrical connection between the two layers 208, 228. The second conductive layer 228 has opposed first 230 and second 232 surfaces with the first surface 230 facing the second surface 214 of the first conductive layer 208.

The printing process of step 308 may be repeated multiple times, by multiple passes of the pressure-driven extrusion printer, to form multiple layers of the second conductive material to build up the second conductive layer 228 and form the electrode 116. The deposition of the second conductive material may be controlled by controlling the number of passes, varying the speed, and/or varying the pressure of the printer as previously described.

In an embodiment, the method includes the step of forming a second conductive ink including the second conductive material disposed in a vehicle. The second conductive material includes electrically conductive particles that are biocompatible. Typically, the electrically conductive particles have a resistivity of less than 1×10⁻⁷ ohm·m. Non-limiting examples of conductive particles include medical grade platinum particles, silver particles, copper particles, gold particles, chromium particles, titanium particles, iridium particles, stainless steel particles, conductive polymers, carbon nanotubes, and combinations thereof. Similar to the first conductive ink described above, the particles have an effective particle diameter of less than 1 µm and may be referred to as nanoparticles. In an embodiment, the particles have an effective particle diameter of from about 50 nm to about 1 µm. Alternatively, the particles could have an effective particle diameter up to about 10 µm. In an embodiment, the second conductive material includes platinum particles, and the second conductive ink may be referred to as a platinum ink. In another embodiment, the platinum particles are optimized by including platinum particles of varying shapes, sizes, and/or concentrations to obtain superior electrical properties such as charge storage capacity, impedance, etc. In an embodiment, the first conductive material of the first conductive ink is the same as the second conductive material of the second conductive ink. Additionally, the shape, size, type, and/or concentration of the first conductive material may be the same or different than that of the second conductive material.

In an embodiment, and similar to the first conductive ink, the vehicle of the second conductive ink is or includes silicone, such as polydimethylsiloxane, which imparts elasticity to the second conductive ink. The silicone is typically used with a curing agent. In another embodiment, the vehicle further includes a siloxane and optionally one or more traditional solvents (such as toluene, heptane, etc.). The siloxane functions to reduce the viscosity of the second conductive ink and tends to evaporate slowly compared to traditional solvents, thereby maintaining ink consistency during printing. Non-limiting examples of suitable siloxanes for the first conductive ink include cyclic siloxanes (such as hexamethylcyclotrisiloxane, octamethylcyclotetrasiloxane, etc.) and linear siloxanes (such as hexamethyldisiloxane, octamethyltrisiloxane, etc.). One or more additives (such as surfactants etc.) may also be used in the second conductive ink. The second conductive ink may be formed in the same way as described above for the first conductive ink.

The resultant electrode assembly 102 is shown in Figure 3F. After the electrode assembly 102 is formed, and in an embodiment, the method further includes the step of depositing a third nonconductive material over the electrode assembly 102. This third nonconductive layer operates to seal and electrically isolate the electrical connection formed between the connector (to be disposed in the aligned apertures 230, 232 (and 234)) and the electrodes 116 of the electrode array 102.

Steps 304 through 308 (in connection with Figures 3C through 3E) are described above for forming a single electrode 116. Additional electrodes may be formed in the same manner, typically simultaneously with the formation of the electrode 116 described above. For example, several first conductive layers 208 may be deposited (by printing) along the second surface 206 of the first insulating layer 202 with adjacent layers 208 spaced from one another. The first conductive layers 208 may be deposited by any desired pattern, such as aligned in one or more rows. The electrodes 116 are formed or built by deposition of the second conductive layers 218 deposited over the respective first conductive layers 208 according to the pattern. An example of this is shown in Figure 2, where the electrodes 116 are formed in a single row. The second insulating layer 218 is deposited in the same manner as previously described to embed the relevant portion(s) of each of the first conductive layers 208 and electrically isolate the individual electrodes 116 that are subsequently formed. The depositing of the second insulating layer 218 to form the several electrodes 116 is accomplished simultaneously.

Once the electrodes 116 are formed, the electrode array 102 is removed from the mold. The resultant electrode array 102 is shown in Figure 3F. In instances where a support 23 is formed, the method further includes the step of removing the support 203 from the second surface 204 of the first substrate layer 202 to form the electrode array 102. The support 203 may be removed utilizing any suitable removal technique, such as peeling, laser cutting, etching, etc.

In an embodiment, the layers 202, 208, 218, 228 are cured independently during formation of each individual layer 202, 208, 218, 228 utilizing the printer. Alternatively, the entire electrode array 102 may be cured after the individual layers 202, 208, 218, 228 have been formed. In this alternative embodiment, curing of the electrode array 102 may be accomplished at a temperature of, for example, up to about 160°C utilizing a hotplate or an oven.

Although not shown, several layers of conductive features separated by insulating layers may be deposited (by printing) on top of one another to create a high density of electrode contacts and multiple layers of conductive features. In this manner, a network of conductive features may be formed to minimize the device footprint and maximize functionality.

Additionally, a plurality of electrode arrays 102 may be formed by repeating the method described in detail above. For example, the plurality of electrode arrays 102 are formed by repeating each of the forming step (step 302) and the depositing steps (steps 304, 306, and 308). Once formed, the individual electrode arrays 102 may then be attached to a pre-stretched elastomeric substrate.

Embodiments of the manufacturing method described in detail above may be used to form an electrode array that is flexible and elastic such that the electrode array 102 can be used for neuroprosthetic devices to be implanted or used in small curved areas or spaces. The method advantageously utilizes pressure-driven extrusion printing, is a direct write printing technique, to form a 3D construct including individual material layers containing silicone to form the electrode array that can flex, bend, or be pulled. The pressure-driven extrusion printing technique also enables formation of small 3D features in one or more of the individual material layers of the electrode array.

The electrode array 102 formed by the method of the present disclosure is advantageously flexible and elastic such that the electrode array 102 can be used for stimulating devices, such as neuroprosthetic devices, to be implanted or used in curved areas or spaces. In an embodiment, the electrode array 102 is stretchable for up to about 140% elongation while remaining electrically conductive. The stretchability of the electrode array 102 is measured using a tensile test where the electrode array 102 is immersed in saline for 10 days at 37°C and removed to undergo a percent (%) elongation at 1 mm/s followed by a 1-minute hold before returning to the relaxed state. The electrode array 102 was stretched until no electrical resistance could be recorded. The electrode array 102 also passes the 15 degree flex test (where the array 102 was flexed 15 degrees at 2 Hz around a 2mm rod while undergoing a 0.03N force for 100,000 cycles), a 90 degree bend test (where the array 102 was bent 90 degrees around a 5mm rod at 1 mm/s for 10 cycles), and a 360 degrees torsion test (where the array 102 was twisted 360 degrees forward before reversing 360 degrees at 1 mm/s for 50 cycles). Based, at least on these results, the electrode array 102 complies with European Standard EN 45502 parts 1 and 2 and with American National Standard ANSI/AAMI C186:2017 for cochlear implant systems.

Additionally, the electrodes 116 of the electrode array 102 exhibit a cathodic charge storage capacity of from about 30 to about 200 mC/cm², and an electrochemical impedance spectroscopy (EIS) of from about 100 to about 5000'Q at 1 kHz for a line thickness of from about 25 to about 300 µm. The electrodes 116 (formed from the first and second conductive inks including at least conductive particles and silicone) exhibits a lower polarization compared to Platinum foil electrodes. This lower voltage potential indicates that a higher charge density may be inputted without causing tissue damage to the organism.

## Claims

1. A method of manufacturing an electrode array (102), said method comprising the steps of:
forming a first insulating layer (202) from a first nonconductive material with said first insulating layer having opposed first and second surfaces (204, 206);
depositing, by pressure-driven extrusion printing, a first conductive material over a portion of the second surface of the first insulating layer to form a first conductive layer (208) having opposed first and second surfaces (212, 214) with the first surface of the first conducting layer facing the first insulating layer, with another portion of the second surface (217) of the first insulating layer remaining exposed;
depositing a second nonconductive material over a portion of the second surface of the first conductive layer and over the exposed portion of the second surface of the first insulating layer to form a second insulating layer (218) having opposed first and second surfaces (220, 222) and a gap (224) extending between the first and second surfaces of the second insulating layer with the gap exposing a portion of the second surface of the first conductive layer; and
depositing, by pressure-driven extrusion printing, a second conductive material into the gap and over the exposed portion of the second surface of the first conductive layer to form a second conductive layer (228) electrically connected to the first conductive layer to form at least one electrode (116) of the electrode array

2. The method as set forth in claim 1 wherein each of the steps of depositing the first nonconductive material and depositing the second nonconductive material is performed by printing.

3. The method as set forth in claim 1 or claim 2, wherein the step of depositing the firs nonconductive material includes the steps of:
depositing a first portion of the first nonconductive material to form a support; and then
depositing a second portion of the first nonconductive material over the deposited first portion by printing to form the first insulating layer.

4. The method as set forth in claim 3 wherein the step of depositing the second portion of the first nonconductive material over the deposited first portion by printing is accomplished utilizing a pressure-driven extrusion printer operating at a rate of about 1 to about 50 mm/s and at a pressure of less than 100 psi.

5. The method as set forth in claim 3 or claim 4 further comprising the step of curing the first and second portions of the first nonconductive material during or after the step of depositing the second portion of the first nonconductive material to solidify the support and the first insulating layer.

6. The method as set forth in any of claims 3 to 5 wherein after the step of depositing the second conductive material, the method further comprises the step of removing the support from the electrode array.

7. The method as set forth in any preceding claim, wherein the step of depositing the first conductive material includes forming the first conductive layer having a plurality of first three-dimensional conductive features.

8. The method as set forth in any preceding claim, wherein the step of depositing the first conductive material is performed utilizing a pressure-driven extrusion printer and includes the step of depositing the first conductive material onto the second surface of the first insulating layer with multiple passes, varying speeds, varying pressures, or combinations thereof of the pressure-driven extrusion printer to form the first conductive layer having multiple thicknesses defining a plurality of first three-dimensional conductive features.

9. The method as set forth in any preceding claim, further comprising the step of forming a first conductive ink including the first conductive material disposed in a vehicle including silicone, and the step of depositing the first conductive material is further defined as depositing the first conductive ink over the portion of the second surface of the first insulating layer to form the first conductive layer, and
further comprising the step of forming a second conductive ink including the second conductive material disposed in a vehicle including silicone, and the step of depositing the second conductive material is further defined as depositing the second conductive ink into the gap and over the exposed portion of the second surface of the first conductive layer to form the second conductive layer electrically connected to the first conductive layer, and
wherein the first conductive material of the first conductive ink is the same as the second conductive material of the second conductive ink.

10. The method as set forth in claim 9 wherein during the step of depositing the first conductive ink, the method includes the step of varying a particle density, a viscosity, or combinations thereof of the first conductive ink to form the first conductive layer having multiple thicknesses defining a plurality of first three-dimensional conductive features.

11. The method as set forth in any preceding claim, further comprising the step of patterning all but the exposed portion of the second surface of the first conductive layer to form a plurality of three-dimensional conductive features.

12. The method as set forth in any preceding claim, wherein each of the steps of depositing the first and second conductive materials is performed utilizing a pressure-driven extrusion printer having a plurality of nozzles with one of the plurality of nozzles configured to deposit the first and second conductive materials.

13. The method as set forth in any preceding claim, further comprising the steps of:
forming at least one aperture in the first insulating layer; and
forming at least one aperture in the second insulating with the at least one aperture of the second insulating layer aligned with the at least one aperture of the first insulating layer,
wherein the aligned apertures are adapted to receive a feedthrough pin of a stimulator of a neuroprosthetic device.

14. The method as set forth in any preceding claim, wherein the electrode array is further defined as a plurality of electrode arrays and further comprising the step of repeating each of the forming step and the depositing steps to form the plurality of electrode arrays.

15. The method as set forth in any preceding claim, wherein the at least one electrode is stretchable up to about 140% elongation while remaining electrically conductive.

## Patentansprüche

1. Verfahren zum Herstellen einer Elektrodengruppierung (102), wobei das Verfahren die folgenden Schritte umfasst:
Bilden einer ersten isolierenden Schicht (202) aus einem ersten nichtleitfähigen Material, wobei die erste isolierende Schicht eine erste und eine entgegengesetzte zweite Oberfläche (204, 206) aufweist,
Absetzen, durch druckgetriebenes Extrusionsdrucken, eines ersten leitfähigen Materials über einen Abschnitt der zweiten Oberfläche der ersten isolierenden Schicht, um eine erste leitfähige Schicht (208) zu bilden, die eine erste und eine entgegengesetzte zweite Oberfläche (212, 214) aufweist, wobei die erste Oberfläche der ersten leitfähigen Schicht der ersten isolierenden Schicht gegenüberliegt, wobei ein anderer Abschnitt der zweiten Oberfläche (217) der ersten isolierenden Schicht freigelegt bleibt,
Absetzen eines zweiten nichtleitfähigen Materials über einen Abschnitt der zweiten Oberfläche der ersten leitfähigen Schicht und über den freigelegten Abschnitt der zweiten Oberfläche der ersten isolierenden Schicht, um eine zweite isolierende Schicht (218) zu bilden, die eine erste und eine entgegengesetzte zweite Oberfläche (220, 222) und einen Spalt (224), der sich zwischen der ersten und der zweiten Oberfläche der zweiten isolierenden Schicht erstreckt, aufweist, wobei der Spalt einen Abschnitt der zweiten Oberfläche der ersten leitfähigen Schicht freilegt, und
Absetzen, durch druckgetriebenes Extrusionsdrucken, eines zweiten leitfähigen Materials in den Spalt und über den freigelegten Abschnitt der zweiten Oberfläche der ersten isolierenden Schicht, um eine zweite leitfähige Schicht (228) zu bilden, die elektrisch mit der ersten leitfähigen Schicht verbunden ist, um mindestens eine Elektrode (116) der Elektrodengruppierung zu bilden.

2. Verfahren nach Anspruch 1, wobei jeder der Schritte des Absetzens des ersten nichtleitfähigen Materials und des Absetzens des zweiten nichtleitfähigen Materials durch Drucken durchgeführt wird.

3. Verfahren nach Anspruch 1 oder Anspruch 2, wobei der Schritt des Absetzens des ersten nichtleitfähigen Materials die folgenden Schritte einschließt:
Absetzen eines ersten Teils des ersten nichtleitfähigen Materials, um einen Träger zu bilden, und danach
Absetzen eines zweiten Teils des ersten nichtleitfähigen Materials über den abgesetzten ersten Teil durch Drucken, um die erste isolierende Schicht zu bilden.

4. Verfahren nach Anspruch 3, wobei der Schritt des Absetzens des zweiten Teils des ersten nichtleitfähigen Materials über den abgesetzten ersten Teil durch Drucken unter Benutzung eines druckgetriebenen Extrusionsdruckers ausgeführt wird, der mit einer Geschwindigkeit von etwa 1 bis etwa 50 mm/s und mit einem Druck von weniger als 100 psi arbeitet.

5. Verfahren nach Anspruch 3 oder Anspruch 4, das ferner den Schritt des Aushärtens des ersten und des zweiten Teils des ersten nichtleitfähigen Materials während des Schrittes des Absetzens des zweiten Teils des ersten nichtleitfähigen Materials oder nach demselben umfasst, um den Träger und die erste isolierende Schicht fest werden zu lassen.

6. Verfahren nach einem der Ansprüche 3 bis 5, wobei nach dem Schritt des Absetzens des zweiten leitfähigen Materials das Verfahren ferner den Schritt des Entfernens des Trägers von der Elektrodengruppierung umfasst.

7. Verfahren nach einem der vorhergehenden Ansprüche, wobei der Schritt des Absetzens des ersten leitfähigen Materials das Bilden der ersten leitfähigen Schicht einschließt, die eine Vielzahl von ersten dreidimensionalen leitfähigen Merkmalen aufweist.

8. Verfahren nach einem der vorhergehenden Ansprüche, wobei der Schritt des Absetzens des ersten leitfähigen Materials unter Benutzung eines druckgetriebenen Extrusionsdruckers durchgeführt wird und den Schritt des Absetzens des ersten leitfähigen Materials auf die zweite Oberfläche der ersten isolierenden Schicht mit mehreren Durchgängen, sich verändernden Geschwindigkeiten, sich verändernden Drücken oder Kombinationen derselben des druckgetriebenen Extrusionsdruckers einschließt, um die erste leitfähige Schicht zu bilden, die mehrere Dicken aufweist, die eine Vielzahl von ersten dreidimensionalen leitfähigen Merkmalen definieren.

9. Verfahren nach einem der vorhergehenden Ansprüche, das ferner den Schritt des Bildens einer ersten leitfähigen Farbe umfasst, die das erste leitfähige Material einschließt, das in einer Trägersubstanz verteilt ist, die Silikon einschließt, und wobei der Schritt des Absetzens des ersten leitfähigen Materials ferner definiert ist als ein Absetzen der ersten leitfähigen Farbe über den Abschnitt der zweiten Oberfläche der ersten isolierenden Schicht, um die erste leitfähige Schicht zu bilden, und
das ferner den Schritt des Bildens einer zweiten leitfähigen Farbe umfasst, die das zweite leitfähige Material einschließt, das in einer Trägersubstanz verteilt ist, die Silikon einschließt, und wobei der Schritt des Absetzens des zweiten leitfähigen Materials ferner definiert ist als ein Absetzen der zweiten leitfähigen Farbe in den Spalt und über den freigelegten Abschnitt der zweiten Oberfläche der ersten leitfähigen Schicht, um die zweite leitfähige Schicht zu bilden, die elektrisch mit der ersten leitfähigen Schicht verbunden ist, und
wobei das erste leitfähige Material der ersten leitfähigen Schicht dasselbe ist wie das zweite leitfähige Material der zweiten leitfähigen Schicht.

10. Verfahren nach Anspruch 9, wobei während des Schrittes des Absetzens der ersten leitfähigen Farbe das Verfahren den Schritt des Veränderns einer Teilchendichte, einer Viskosität oder von Kombinationen derselben der ersten leitfähigen Farbe einschließt, um die erste leitfähige Schicht zu bilden, die mehrere Dicken aufweist, die eine Vielzahl von ersten dreidimensionalen leitfähigen Merkmalen definieren.

11. Verfahren nach einem der vorhergehenden Ansprüche, das ferner den Schritt des Musterns von allem außer dem freigelegten Abschnitt der zweiten Oberfläche der ersten leitfähigen Schicht umfasst, um eine Vielzahl von dreidimensionalen leitfähigen Merkmalen zu bilden.

12. Verfahren nach einem der vorhergehenden Ansprüche, wobei jeder der Schritte des Absetzens des ersten und des zweiten leitfähigen Materials unter Benutzung eines druckgetriebenen Extrusionsdruckers durchgeführt wird, der eine Vielzahl von Düsen aufweist, wobei eine von der Vielzahl von Düsen dafür konfiguriert ist, das erste und das zweite leitfähige Material abzusetzen.

13. Verfahren nach einem der vorhergehenden Ansprüche, das ferner die folgenden Schritte umfasst:
Formen mindestens einer Öffnung in der ersten isolierenden Schicht und
Formen mindestens einer Öffnung in der zweiten isolierenden Schicht, wobei die mindestens eine Öffnung der zweiten isolierenden Schicht mit der mindestens einen Öffnung der ersten isolierenden Schicht ausgerichtet ist,
wobei die ausgerichteten Öffnungen angepasst sind, um einen Durchführungsstift eines Stimulators einer neuroprothetischen Vorrichtung aufzunehmen.

14. Verfahren nach einem der vorhergehenden Ansprüche, wobei die Elektrodengruppierung ferner als eine Vielzahl von Elektrodengruppierungen definiert ist, und das ferner den Schritt des Wiederholens sowohl des Bildungsschrittes als auch der Absetzschritten umfasst, um die Vielzahl von Elektrodengruppierungen zu bilden.

15. Verfahren nach einem der vorhergehenden Ansprüche, wobei die mindestens eine Elektrode bis zu etwa 140 % Längung dehnbar ist, während sie elektrisch leitfähig bleibt.

## Revendications

1. Procédé de fabrication d'un réseau d'électrodes (102), ledit procédé comprenant les étapes de :
formation d'une première couche isolante (202) dans un premier matériau non-conducteur avec ladite première couche isolante ayant des première et seconde surfaces opposées (204 ; 206) ;
de dépose, par impression par extrusion par pression, d'un premier matériau conducteur au-dessus d'une partie de la seconde surface de la première couche isolante pour former une première couche conductrice (208) ayant des première et seconde surfaces opposées (212, 214) avec la première surface de la première couche conductrice faisant face à la première couche isolante, avec une autre partie de la seconde surface (217) de la première couche isolante restant exposée ;
de déposer un second matériau non-conducteur au-dessus d'une partie de la seconde surface de la première couche conductrice et au-dessus de la partie exposée de la seconde surface de la première couche isolante pour former une seconde couche isolante (218) ayant des première et seconde surfaces opposées (220, 222) et un espace (224) s'étendant entre les première et seconde surfaces de la seconde couche isolante avec l'espace exposant une partie de la seconde surface de la première couche conductrice ; et
de dépose, par impression par extrusion par pression, d'un second matériau conducteur dans l'espace et au-dessus de la partie exposée de la seconde surface de la première couche conductrice pour former une seconde couche conductrice (228) connectée électriquement à la première couche conductrice pour former au moins une électrode (116) du réseau d'électrodes.

2. Procédé selon la revendication 1, dans lequel chacune des étapes de dépôt du premier matériau non-conducteur et de dépôt du second matériau non-conducteur est exécutée par impression.

3. Procédé selon la revendication 1 ou la revendication 2, dans lequel l'étape de dépôt du premier matériau non-conducteur inclut les étapes de :
déposer une première partie du premier matériau non-conducteur pour former un support ; puis de
déposer une seconde partie du premier matériau non-conducteur au-dessus de la première partie déposée par impression pour former la première couche isolante.

4. Procédé selon la revendication 3, dans lequel l'étape de déposer la seconde partie du premier matériau non-conducteur au-dessus de la première partie déposée par impression est réalisée en utilisant une imprimante à extrusion par pression fonctionnant à une vitesse d'environ 1 à environ 50 mm/s et à une pression inférieure à 100 psi.

5. Procédé selon la revendication 3 ou la revendication 4, comprenant en outre l'étape de sécher les première et seconde parties du premier matériau non-conducteur pendant ou après l'étape de dépôt de la seconde partie du premier matériau non-conducteur pour solidifier le support et la première couche isolante.

6. Procédé selon l'une quelconque des revendications 3 à 5, dans lequel après l'étape de déposer le second matériau conducteur, le procédé comprend en outre l'étape de retirer le support du réseau d'électrodes.

7. Procédé selon l'une quelconque revendication précédente, dans lequel l'étape de déposer le premier matériau conducteur inclut de former la première couche conductrice ayant une pluralité de premières caractéristiques conductrices tri-dimensionnelles.

8. Procédé selon l'une quelconque revendication précédente, dans lequel l'étape de déposer le premier matériau conducteur est réalisée en utilisant une imprimante à extrusion par pression et inclut l'étape de déposer le premier matériau conducteur sur la seconde surface de la première couche isolante avec des passes multiples, vitesses qui varient, pressions qui varient ou des combinaisons de celles-ci de l'imprimante à extrusion par pression pour former la première couche conductrice ayant des épaisseurs multiples définissant une pluralité de premières caractéristiques conductrices tri-dimensionnelles.

9. Procédé selon l'une quelconque revendication précédente, comprenant en outre l'étape de former une première encre conductrice incluant le premier matériau conducteur déposé dans un liant incluant de la silicone et l'étape de déposer le premier matériau conducteur est en outre définie en tant que le dépôt de la première encre conductrice au-dessus de la partie de la seconde surface de la première couche isolante pour former la première couche conductrice, et
comprenant en outre l'étape de former une seconde encre conductrice incluant le second matériau conducteur déposé dans un liant incluant de la silicone, et l'étape de déposer le second matériau conducteur est en outre définie en tant que le dépôt de la seconde encre conductrice dans l'espace et au-dessus de la partie exposée de la seconde surface de la première couche conductrice pour former la seconde couche conductrice connectée électriquement à la première couche conductrice, et
dans lequel la première couche conductrice de la première encre conductrice est la même que le second matériau conducteur de la seconde encre conductrice.

10. Procédé selon la revendication 9, dans lequel durant l'étape de dépôt de la première encre conductrice, le procédé inclut l'étape de faire varier une densité de particules, une viscosité ou des combinaisons de celles-ci de la première encre conductrice pour former la première couche conductrice ayant des épaisseurs multiples définissant une pluralité de premières caractéristiques conductrices tri-dimensionnelles.

11. Procédé selon l'une quelconque revendication précédente, comprenant en outre l'étape de réaliser un tracé de tout sauf la partie exposée de la seconde surface de la première couche conductrice pour former une pluralité de caractéristiques conductrices tri-dimensionnelles.

12. Procédé selon l'une quelconque revendication précédente, dans lequel chacune des étapes de dépôt des premier et second matériaux conducteurs est exécutée en utilisant une imprimante à extrusion par pression ayant une pluralité de buses avec une de la pluralité de buses configurée pour déposer les premier et second matériaux conducteurs.

13. Procédé selon l'une quelconque revendication précédente, comprenant en outre les étapes de :
former au moins une ouverture dans la première couche isolante ; et
former au moins une ouverture dans la seconde couche isolante avec l'au moins une ouverture de la seconde couche isolante alignée avec l'au moins une ouverture de la première couche isolante,
dans lequel les ouvertures alignées sont adaptées pour recevoir une broche de connexion d'un stimulateur d'un dispositif neuroprothétique.

14. Procédé selon l'une quelconque revendication précédente, dans lequel le réseau d'électrodes est en outre défini en tant qu'une pluralité de réseaux d'électrodes et comprenant en outre l'étape de répéter chacune de l'étape de formation et des étapes de dépôt pour former la pluralité de réseaux d'électrodes.

15. Procédé selon l'une quelconque revendication précédente, dans lequel l'au moins une électrode est étirable jusqu'à environ 140 % d'allongement tout en restant électriquement conductrice.
